# EUROPEAN PATENT SPECIFICATION

(11) **EP 1 660 029 B1**
(45) Date of publication and mention of the grant of the patent: **18.03.2015**
(21) Application number: 04765608.7
(22) Date of filing: 23.08.2004
(51) Int. Cl.: A61K 8/34, A61Q 5/02, A61K 8/63, A61Q 19/00, A61K 47/10, A61K 31/56, A61K 9/00

(54) **USE OF PENTYLENE GLYCOL AS SOLVENT FOR HYDROCORTISONE OR DERIVATIVES THEREOF**
VERWENDUNG VON PENTYLENGLYCOL ALS LÖSUNGSMITTEL FÜR HYDROCORTISON ODER HYDROCORTISON-DERIVATE
UTILISATION DE PENTYLÈNE GLYCOL COMME SOLVANT POUR HYDROCORTISONE OU SES DÉRIVÉS

(30) Priority: 22.08.2003 US 646300
(43) Date of publication of application: 31.05.2006
(73) Proprietor: L'Oréal, 75008 Paris (FR)
(72) Inventor: HANSENNE, Isabelle, Westfield, NJ 07090 (US); FARES, Hani, Somerset, NJ 08873 (US); CORNELL, Marc, Jackson, NJ 08527 (US); FOLTIS, Peter, Nutley, NJ 07110 (US)
(74) Representative: Jupin, Claude Christian Marie
(86) International application number: PCT/EP2004/010771
(87) International publication number: WO 2005/018582

(56) References cited:
- EP-A- 0 321 951
- EP-A- 1 078 638
- WO-A-03/011244
- WO-A-2005/004830
- DATABASE CA [Online] CHEMICAL ABSTRACTS SERVICE, COLUMBUS, OHIO, US; 28 February 2002 (2002-02-28), FUKUDA, YASUHIRO ET AL: "Skin preparations containing 1,2-pentanediol as percutaneous absorption enhancer" XP002326555 retrieved from STN Database accession no. 2002:235862 -& JP 2002 087926 A (POLA CHEMICAL INDUSTRIES, INC., JAPAN) 27 March 2002 (2002-03-27)

## Description

### BACKGROUND OF THE INVENTION

Hydrocortisone is used in many topical preparations as a treatment for temporary relief of itching associated with minor skin irritation, inflammation and rashes due to eczema, insect bites, poison ivy, poison oak, poison sumac, soaps, detergents, cosmetics, seborrheic dermatitis, psoriasis and itching in the genital and anal areas of the body. Hydrocortisone has limited solubility in water. Thus, it is necessary to add co-solvents, surfactants, and/or complexing agents to obtain an aqueous solution of hydrocortisone in sufficient concentration to be therapeutically efficacious.

WO03/011244 discloses enhancing the solubility of Dehydroepiandrosterone (DHEA) in water by the use of dipropylene glycol.

U.S. Patent 2,880,130 discloses the use of polyoxyethylene sorbitan monooleate (Tween 80^{®}) in amounts of from 2-25 percent of the vehicle to obtain clear aqueous solutions containing up to 0.2% of hydrocortisone. U.S. Patent 4,289,764 describes formulations containing 0.025 to 0.4% hydrocortisone in an aqueous solution of 15-50% propylene glycol that is acidified to pH 2.7-3.3 with a non-toxic organic acid such as citric acid. U.S. Patent 4,305,936 provides for a 0.005-2.5% hydrocortisone clear liquid formulation containing 1-4% by weight of a glyceryl ester of fatty acids having 6-22 carbon atoms, 1-3% by weight of the hydrocortisone of a betaine surfactant, and 10-50% of an alkanol co-solvent, preferably ethanol. U.S. Patent 4,778,060 describes a 0.5% hydrocortisone aqueous solution for use as a douche and for impregnating towelettes for wipes. The solution also contains caprylic/capric triglycerides (5-20%), sorbitan stearate (2-4%), Polysorbate 60^{®} (1-3%), preservatives and citric acid.

U.S. Patent 4,383,992 discloses an aqueous solution of an inclusion complex of unbranched beta-cyclodextrin and hydrocortisone and reveals that the inclusion complex must dissociate before the hydrocortisone is physiologically active. U.S. Patent 5,229,370 discloses an aqueous solution on an inclusion complex composed of a branched beta-cyclodextrin and hydrocortisone. U.S. Patent 4,853,379 teaches hydrocortisone compositions containing a mixture of solvents which are an aliphatic alcohol, propylene glycol and dimethyl coco-benzylammonium chloride. U.S. Patent 4,971,789 teaches various ionic polyethers as solubilizers for pharmaceuticals such as hydrocortisone. U.S. Patent 5,190,936 teaches compositions containing hydrocortisone and a lipid phase of nonionic amphiphilic lipid vesicles. GB 2,131,693 teaches hydrocortisone compositions containing a solvent mixture of (i) a caprolactam and (ii) 2-isostearyl-1-hyroxylethyl-1-benzylimidazolinium chloride or an alkylphenol polyglycerol. WO 96/20712 teaches aqueous solutions of hydrocortisone free of lower alcohols that contain sodium dioctyl sulfosuccinate in mixtures of glycerin, propylene glycol and polyethylene glycol.

### SUMMARY OF THE INVENTION

A first aspect of the present invention is directed to the use of pentylene glycol as solvent for hydrocortisone or derivatives thereof chosen amongst hydrocortisone 21-acetate, hydrocortisone 21-benzadac, hydrocortisone 21-cyclopentyl propionate, hydrocortisone 21-hemisuccinate, hydrocortisone 21-acetate 17-propionate, hydrocortisone 17-butyrate, hydrocortisone 17-valerate and hydrocortisone 17-butyrate 21-propionate, in a cosmetic or dermatological composition for topical use, wherein the composition comprises ar least one solvent other than pentylene glycol.

The present invention describes a cosmetic or dermatological composition for topical use, comprising a steroidal hormone or anti-inflammatory agent, and a solvent for the hormone or anti-inflammatory agent comprising pentylene glycol.

A second aspect of the present invention is directed to a cosmetic or dermatological composition for topical use, comprising at least hydrocostisone or derivatives thereof chosen amongst hydrocortisone 21-acetate, hydrocortisone 21-benzadac, hydrocortisone 21-cyclopentyl propionate, hydrocortisone 21-hemisuccinate, hydrocortisone 21-acetate 17-propionate, hydrocortisone 17-butyrate, hydrocortisone 17-valerate and hydrocortisone 17-butyrate 21-propionate, pentylene glycol as solvent of said corticosteroid and at least one solvent other than pentylene glycol, the amount of said hydrocortisone or derivatives thereof being of between 0.5% and 12% by weight of said composition.

The present invention describes a method of formulating a steroidal hormone or anti-inflammatory agent for use in a cosmetic or dermatological composition, comprising mixing the steroidal hormone or anti-inflammatory agent and pentylene glycol.

A third aspect of the present invention is directed to a method of formulating hydrocortisone or derivatives thereof chosen amongst hydrocortisone 21-acetate, hydrocortisone 21-benzadac, hydrocortisone 21-cyclopentyl propionate, hydrocortisone 21-hemisuccinate, hydrocortisone 21-acetate 17-propionate, Hydrocortisone 17-butyrate, hydrocortisone 17-valerate and hydrocortisone 17-butyrate 21-propionate, for use in a cosmetic or dermatological composition, comprising mixing said hydrocortisone or derivatives thereof and a solvent for hydrocortisone or derivatives thereof comprising pentylene glycol and at least one other solvent than pentylene glycol, the amount of said hydrocortisone or derivatives thereof being of between 0.5% and 12%µ by weight of said composition

The present invention describes a method of treating skin or scalp, comprising applying to skin or scalp a composition comprising hydrocortisone or derivatives thereof chosen amongst hydrocortisone 21-acetate, hydrocortisone 21-benzadac, hydrocortisone 21-cyclopentyl propionate, hydrocortisone 21-hemisuccinate, hydrocortisone 21-acetate 17-propionate, hydrocortisone 17-butyrate, hydrocortisone 17-valerate and hydrocortisone 17-butyrate 21-propionate and pentylene glycol.

The compositions of the present invention may be formulated in a variety of ways, e.g., as a gel, emulsion, ointment, shampoo or lotion. They may be applied to skin or scalp for cosmetic or dermatological purposes e.g., for conditions amenable to treatment with the steroidal hormone or anti-inflammatory agents.

Applicants have discovered that hydrocortisone and the above indicated derivatives, are more soluble in pentylene glycol than other polyols and its homologs such as glycerol, propylene glycol, butylene glycol and hexylene glycol. Accordingly, compositions of the present invention may contain less total solvent compared to current products. Compositions of the present invention thus offer more aesthetic appeal. Since steroidal hormones and anti-inflammatory agents must be in solution to permeate skin, the compositions may also offer greater efficacy from the standpoint of delivery.

### BRIEF DESCRIPTION OF THE DRAWING

Figure 1 is a graph comparing cumulative release of hydrocortisone from various commercial products and an embodiment of the present invention, as a function of time.

### DETAILED DESCRIPTION

Steroid hormone or steroidal anti-inflammatory agent (collectively referred to herein as "active agents") soluble in pentylene glycol (and more soluble in pentylene glycol than in water) and which can be administered transdermally are described in the present invention. Steroid hormones have postmenopausal, anabolic, contraceptive and anti-inflammatory uses. Representative examples include testosterone and dehydroepiandrosterone (DHEA). Examples of steroidal anti-inflammatory agents include fluoracetonide, fludrocortisone, difluorsone diacetate, flurandrenolone acetonide, betamethasone and its other esters, chloroprednisone, desonide, dichlorisone, methylmeprednisolone, cortisone acetate and hydrocortisone cyclopentylpropionate.

Active agents described for use in the present invention are corticosteroids. These are hormonal derivatives that may be prepared synthetically or extracted from the cortex of the adrenal capsules, and which contain a cyclopentanophenanthrene ring-system. They are particularly useful in the treatment of certain skin diseases, such as contact eczema or atopic dermatitis. Corticosteroids include betamethasone, betamethasone dipronionate, beta ethasone phosphate, betamethasone valerate, cortisone, dexamethasone, fludrocortisone, fluocinonide, fluocinonide desonide, fluocinolone, fluocinolone acetonide, fluocortolone, hydrocortisone and its derivatives (e.g., esters thereof such as hydrocortisone 17-valerate, hydrocortisone 17-butyrate and hydrocortisone 21-acetate), methylprednisolone, prednisolone, prednisolone 21-phosphate, prednisone, triamcinolone, triamcinolone acetate and triamcinolone acetonide. More preferred active agents are hydrocortisone and derivatives (e.g. esters e.g., mono- and di-esters, such as hydrocortisone 21-acetate; hydrocortisone 21-benzadac; hydrocortisone 21-cyclopentylpropionate; hydrocortisone 21-hemisuccinate; hydrocortisone 21-acetate 17-propionate; hydrocortisone 17-butyrate; hydrocortisone 17-valerate; and hydrocortisone 17-butyrate 21-propionate.).

The active agent described in the invention is present in the compositions in therapeutically effective amounts. In general, the active agent will be present in the composition in an amount of about 0.01 to about 20% by weight. The amount of hydrocortisone (or derivative thereof) in the compositions according to the present invention is generally not greater than about 12% (e.g., about 0.1 to about 12% by weight). Preferably, the hydrocortisone or derivatives thereof are present in the compositions of the present invention in an amount between 0.01 and 5% by weight, and more particularly between 0.5 and 4%, based on the total weight of the composition. These amounts are effective to treat conditions for which hydrocortisone and its salts and esters are indicated, e.g., eczema, atopic dermatitis, psoriatic or eczematous erythrodermy, pruriginous lesions, chronic erythematous lupus, patch psoriasis and parapsoriasis, hyperthrophic cicatrix, and radiotherapic or solar erythema. Frequency and amount of use of the compositions of the present invention will depend upon numerous factors, including the condition being treated and its severity. For example, application of compositions containing hydrocortisone or derivatives thereof to treat one of the conditions disclosed above typically requires an application of the composition of the present invention to the affected area of the skin, on the average, twice each day, optionally with a massaging action in order to facilitate the penetration thereof into the skin.

Pentylene glycol is typically present in an amount of about 5% to about 70% by weight of the composition. The amount of pentylene glycol used in the composition varies depending on the amount of the active agent and whether it is the only solvent used. For example, applicants have found that the solubility of hydrocortisone in pentylene glycol is about 6%, which as shown in the Examples below, is higher than the solubility of hydrocortisone or a derivative thereof according to the invention in other glycols. Thus, in embodiments where pentylene glycol is the only solvent, and hydrocortisone (or a derivative thereof according to the invention) is present, the pentylene glycol is present in an amount of about 16-17 times the amount of the hydrocortisone or derivative thereof according to the invention. According to the invention, an other solvents (or "co-solvents") other than pentylene glycol, particularly non-volatile solvents, is present in the composition. This solvents includes esters, polyols, such as glycerin, propylene glycol, butylene glycol and hexylene glycol, polyethylene glycols, polypropylene glycols, and mixtures thereof. Additional solvents may be present in an amount of about 5% to about 50% by weight of the composition. In preferred embodiments, pentylene glycol is present in amounts of about 5% to about 25%, and another solvents present in amounts of about 10% to about 70% of the total weight of the composition. Due to the greater solubility of the active agents described in the invention in pentylene glycol, the amounts of the other solvents is significantly lower e.g., about 20 to about 95% less, than if pentylene glycol were not present. Relatively high amounts of glycols are undesirable from several standpoints, especially in terms of aesthetic appeal and tackiness. In contrast, compositions of the present invention are more aesthetically acceptable and have less tackiness.

The compositions according to the present invention can be provided in various forms, principally in the form of emulsions (e.g., creams), gels, lotions and shampoos. They may also be in anhydrous form e.g., in the form of an ointment, gel or pomade. Emulsions and gels are preferred. Compositions of the present invention other than those in anhydrous form have an aqueous phase and an oil or fatty phase. When the composition according to the invention is an emulsion, the proportion of the fatty phase generally ranges from about 0.5% to about 80% by weight and preferably from about 5% to about 50% by weight, based on the total weight of the composition. Oils, waxes, emulsifiers and co-emulsifiers that are typically present in the composition may be selected from those conventionally used in cosmetics and dermatology.

The fatty phase or oily phase usually contains at least one oil. Examples include hydrocarbon-based oils of animal origin, such as perhydrosqualene; hydrocarbon-based oils of plant origin, such as liquid triglycerides of fatty acids containing from 4 to 10 carbon atoms, for instance heptanoic or octanoic acid triglycerides or alternatively, for example, sunflower oil, corn oil, soybean oil, marrow oil, grapeseed oil, sesame oil, hazelnut oil, apricot oil, macadamia oil, arara oil, sunflower oil, castor oil, avocado oil, caprylic/capric acid triglycerides, jojoba oil or karite butter oil; synthetic esters and synthetic ethers, especially of fatty acids, for instance oils of formulae R⁶COOR⁷ and R⁶OR⁷, in which R⁶ represents a fatty acid residue containing from 8 to 29 carbon atoms, and R⁷ represents a branched or unbranched hydrocarbon-based chain containing from 3 to 30 carbon atoms, such as, for example, purcellin oil, isononyl isononanoate, isopropyl myristate, 2-ethylhexyl palmitate, 2-octyl-dodecyl stearate, 2-octyldodecyl erucate or isostearyl isostearate; hydroxylated esters such as isostearyl lactate, octyl hydroxystearate, octyldodecyl hydroxystearate, diisostearyl malate, triisocetyl citrate and fatty alkyl heptanoates, octanoates and decanoates; polyol esters such as propylene glycol dioctanoate, neopentyl glycol diheptanoate and diethylene glycol diisononanoate; and pentaerythritol esters such as pentaerythrityl tetraisostearate; linear or branched hydrocarbons of mineral or synthetic origin, such as volatile or non-volatile liquid paraffins, and derivatives thereof, petroleum jelly, polydecenes, and hydrogenated polyisobutene such as parleam oil; fatty alcohols containing from 8 to 26 carbon atoms, such as cetyl alcohol, stearyl alcohol and a mixture thereof (cetylstearyl alcohol), octyldodecanol, 2-butyloctanol, 2-hexyldecanol, 2-undecylpentadecanol, oleyl alcohol or linoleyl alcohol; alkoxylated and ethoxylated fatty alcohols such as oleth-12; partially hydrocarbon-based and/or silicone-based fluoro oils such as perfluoromethylcyclopentane, perfluoro-1,3-dimethylcyclohexane, perfluoro-1,2-dimethylcyclo-butane; perfluoroalkanes such as dodecafluoropentane and tetradecafluorohexane, bromoperfluorooctyl; nonafluoromethoxybutane and nonafluoro-ethoxyisobutane; perfluoromorpholine derivatives, such as 4-trifluoromethylperfluoromorpholine; silicone oils such as volatile or non-volatile polymethylsiloxanes (PDMSs) containing a linear or cyclic silicone chain, and that are liquid or pasty at room temperature, e.g., cyclopolydimethylsiloxanes (cyclomethicones) such as cyclohexa-siloxane; polydimethylsiloxanes comprising alkyl, alkoxy or phenyl groups, that are pendent or at the end of a silicone chain, these groups containing from 2 to 24 carbon atoms; phenylsilicones, such as phenyltrimethicones, phenyldimethicones, phenyltrimethylsiloxydiphenylsiloxanes, diphenyldimethicones, diphenylmethyldiphenyltrisiloxanes, 2-phenylethyl-trimethylsiloxysilicates and polymethylphenylsiloxanes; and mixtures thereof.

Other fatty substances that may be present in the oily phase are, for example, fatty acids containing from 8 to 30 carbon atoms, such as stearic acid, lauric acid, palmitic acid and oleic acid; waxes of animal origin, such as lanolin, beeswax, spermaceti or lanolin derivatives, such as lanolin alcohols, hydrogenated, hydroxylated or acetylated lanolin, lanolin fatty acids and acetylated lanolin alcohol; waxes of vegetable origin, such as carnauba, candelilla, kapok, ouricury, rice, hydrogenated jojoba, esparto or japan wax or cork fibre or sugar cane waxes or cocoa butter; mineral waxes, for example paraffin, montan, lignite or petrolatum waxes or microcrystalline waxes, ceresin or ozokerite; or synthetic waxes, such as polyethylene waxes, waxes obtained by the Fischer-Tropsch synthesis and linear esters resulting from the reaction of a saturated C₁₀ to C₄₀ carboxylic acid and of a saturated C₁₀ to C₄₀ alcohol, such as myristyl myristate. Use may also be made of cetyl alcohol, stearyl alcohol, calcium lanolates or stearates, castor oil, palm oil, coconut oil, sunflower oil or hydrogenated coconut oil; gums such as silicone gums (dimethiconol); silicone resins such as trifluoromethyl-C1-4-alkyldimethicone and trifluoropropyldimethicone; and silicone elastomers. These fatty substances may be chosen in a varied manner to prepare a composition having the desired properties, e.g., consistency or texture.

Emulsions may contain at least one emulsifier e.g., amphoteric, anionic, cationic and nonionic emulsifiers, used alone or as a mixture. Choice of emulsifier depends upon the nature of the emulsion e.g., water-in-oil (W/O) or oil-in-water (O/W) emulsions. Examples of emulsifiers that may be used in O/W emulsions include nonionic emulsifiers such as saccharide esters and ethers such as sucrose stearate, sucrose cocoate (and mixtures of sucrose stearate and cocoate); polyol esters, in particular glycerol or sorbitol esters, such as glyceryl stearate, polyglyceryl-2 stearate and sorbitan stearate; glycerol ethers; oxyethylenated and/or oxypropylenated ethers such as the oxyethylenated, oxypropylenated ether of lauryl alcohol containing 25 oxyethylene groups and 25 oxypropylene groups (CTFA name "PPG-25 laureth-25") and the oxyethylenated ether of the mixture of C₁₂-C₁₅ fatty alcohols containing 7 oxyethylene groups (CTFA name " C₁₂-C₁₅ Pareth-7"); ethylene glycol polymers such as PEG-100, and mixtures thereof.

Examples of emulsifiers for use in W/O emulsions include fatty esters of a polyol, in particular of glycerol or of sorbitol, and in particular polyol isostearates, oleates and ricinoleates; saccharide esters and ethers such as methyl glucose dioleate; fatty esters such as magnesium lanolate; dimethicone copolyols and alkyldimethicone copolyols.

Examples of surfactants (emulsifying and coemulsifying) include the esters of fatty acids and polyethylene glycol (PEG), esters of fatty acids and glycerol (glyceryl stearate) or esters of fatty acids and sugar (sorbitan stearate), as well as the polyoxyethylenated or polyoxypropylenated derivatives thereof, cyclomethicones and dimethicone copolyols, and also anionic surfactants (e.g., potassium or sodium alkyl phosphates).

Gels are obtained using gelling agents such as cellulose derivatives (e.g., ethyl cellulose), carboxyvinyl polymers (Carbopols), natural or synthetic gums, modified clays (bentones), metal salts of fatty acids (aluminum stearate), ethylene/acrylate copolymers, silicas and polyethylenes. The gelling agent is typically used in an amount of about 0.5 and about 15 %, based on the total weight of the composition.

Lotions generally contain the active agent e.g., hydrocortisone or salt or ester thereof, solubilized in pentylene glycol, optionally with one or more additional solvents. The ointments are anhydrous compositions based, for example, on petrolatum, paraffin oil or waxes.

Other cosmetically or dermatologically acceptable agents that may be used in the compositions of the invention include coloring agents (pigments, dyes, colorants e.g., iron oxides, titanium oxides and zinc oxides), preservatives, perfumes and fragrances, hydrating active agents, ultraviolet ray-absorbing agents (sunscreen or sunblock agents), pulverulent agents, antiperspirants and/or odor absorbers, moisturizers, for example protein hydrolysates and polyols such as glycerol, glycols, for instance polyethylene glycols, and sugar derivatives; natural extracts; procyannidol oligomers; vitamins, for instance vitamin A (retinol), vitamin C (ascorbic acid), vitamin E (tocopherol), vitamin B5 (panthenol) and vitamin B3 (niacinamide); vitamin K; urea; caffeine; depigmenting agents such as kojic acid and caffeic acid; salicylic acid; alpha-hydroxy acids such as lactic acid and glycolic acid; retinoids such as carotenoids; fillers, keratolytic agents, anti-oxidants, melatonin; extracts of algae, fungi, plants, yeasts or bacteria; hydrolysed, partially hydrolysed or unhydrolysed proteins, and enzymes; antibacterial or bactericidal agents e.g., 2,4,4'-trichloro-2'-hydroxydiphenyl ether (triclosan) and 3,4,4'-trichlorocarbanilide (or triclocarban), azelaic acid and benzoylperoxide; matt-effect agents, for instance fibres; tensioning agents; optical brighteners; and mixtures thereof, as well as additional active ingredients aside from the active agents of the present invention. Amounts of such agents typically range from about 0.0001% to about 20% by weight of the composition. For example, U.S. Patent 5,643,898 teaches a combination for inducing and stimulating hair growth, or decreasing hair loss, containing hydrocortisone and a pyrimidine derivative, e.g., Minoxidil. Thus, persons skilled in the art will be able to select additional active ingredients and inert ingredients suitable for administration to skin or scalp as desired. See, e.g., U.S. Patent 5,275,755 (directed to shampoo compositions).

The following examples are intended to further illustrate certain embodiments of the invention and are not intended to limit the invention in any way. Unless otherwise indicated, all percentages are weight-by-weight.

### EXAMPLES

### Example 1: Solubility of Hydrocortisone in Various Solvents

Fifty (50) grams of each of the following solvents were added to a separate beaker, and stirred with a magnetic stirrer at 50 °C. Hydrocortisone (USP from Pharmacia) was added in increments of 0.2 grams to each beaker until maximum solubility was reached. Maximum solubility was determined visually. The results are shown in Table 1. Hydrocortisone was about 3 times more soluble in pentylene glycol than isopropyl lauroyl sarcosinate, about 2 times more soluble in pentylene glycol than hexylene glycol, about 1.5 times more soluble in pentylene glycol than in propylene glycol and about 1.25 times as soluble in pentylene glycol than butylene glycol.

**Table 1**

| | |
|---|---|
| Glycerin: | 0.4% |
| Isopropyl lauroyl sarcosinate: | 2.0% |
| Propylene glycol: | 4.0% |
| Butylene glycol: | 4.8% |
| Pentylene glycol: | 6.0% |
| Hexylene glycol: | 3.2% |

### Example 2: Illustration of Solubilization Capabilities of various Solvents for Hydrocortisone

Water was added to three different solutions containing 1% hydrocortisone. Each contained a different solvent system, i.e., (1) 30 grams of pentylene glycol, (2) 15 grams pentylene glycol and 15 grams butylene glycol, and (3) 10 grams of each of pentylene glycol, butylene glycol and propylene glycol. Water was added until a precipitate was formed. The amount of water was determined for each solution. The results are shown in Table 2. They show that a reduction in amount of pentylene glycol had a negative effect on solubility of hydrocortisone - when the amount of pentylene glycol was reduced by two-thirds, the effectiveness of the solvent system was reduced in half.

**Table 2**

| Amount of wated added (gms) | Solvent system |
|---|---|
| 120 | 30 g pentylene glycol |
| 98 | 15 g pentylene glycol and 15 g butylene glycol |
| 65 | 10 g pentylene glycol, 10 g butylene glycol and 10 g glycol |

### Example 3: Emulsion

**Table 3**

| **Phase** | | **INCI Name (Trade Name)** | |
|---|---|---|---|
| A | | Water | 51.800 |
| | | Propylene glycol | 7.000 |
| | | Pentylene glycol | 10.000 |
| | | Butylene glycol | 10.000 |
| | | Preservatives | 0.300 |
| | | Nylon 12 | 2.000 |
| | | Hydrocortisone, USP (Micronized) | 1.000 |
| | | | |
| B | | Dicaprylyl ether (Cetiol OE) | 3.000 |
| | | C₁₂-₁₅ Alkyl benzoate | 3.000 |
| | | Octyl palmitate | 3.000 |
| | | Isononyl isononaoate | 1.000 |
| | | Cetyl dimethicone (Abil wax 9801) | 1.000 |
| | | Cetearyl alcohol and Dicetyl phosphate and Ceteth-10 phosphate (Crodafos CES) | 3.600 |
| | | Glyceryl monostearate self-emulsifier | 1.500 |
| | | Preservatives | 0.700 |
| C | | Polyacrylamide (and) C13-14 isoparaffin (and) laureth-7 (Sepigel 305) | 1.100 |
| | | Total | 100.00 |

To prepare the composition described in table 3, the batch amount of water was added to the main beaker. The remaining ingredients of phase A were mixed separately and then added to the main beaker. The ingredients of phase B were added to a separate container and mixed at 50° C until clear. The mixture was allowed to cool to room temperature, and then was added to the main beaker while mixing. The mixing was continued until homogeneous. The ingredients of phase C were mixed in a separate container, followed by the addition of the mixture of phases A and B thereto, while mixing. The batch was homogenized for 5 minutes.

### Example 4: Water in Oil Cream

**Table 4**

| Water in oil cream | | | |
|---|---|---|---|
| **Phase** | **INCI Name (Trade Name)** | **%** | **500 G** |
| **A** | Water | 40.20 | 201.00 |
| | Sodium chloride | 0.50 | 2.50 |
| | | | |
| **B** | Propylene glycol | 5.00 | 25.00 |
| | Pentylene glycol | 17.00 | 85.00 |
| | Butylene glycol | 17.00 | 85.00 |
| | Hydrocortisone | 1.00 | 5.00 |
| | | | |
| | | | |
| **C** | Cetyl PEG/PPG-10/1 dimethicone (ABIL EM 90) | 2.30 | 11.50 |
| | Octyl palmitate | 6.50 | 32.50 |
| | Cyclopentasiloxane (GE SF 1214) | 3.50 | 17.50 |
| | Cyclopentasiloxane (Dow Corning DC 245) | 3.50 | 17.50 |
| | C12-15 Alkyl benzoate | 3.50 | 17.50 |
| | | | |
| | | | |
| | Total | | 500.00 |

The batch amount of water was added to the main beaker, followed by addition of sodium chloride while mixing until the sodium chloride was dissolved. The ingredients of phase B were added to a separate container and mixed at 50 °C until clear. The mixture was allowed to cool to room temperature, and then was added to the main beaker while mixing. The mixing was continued until homogeneous. The ingredients of phase C were mixed in a separate container, followed by the addition of the mixture of phases A and B thereto, while mixing. The batch was homogenized for 5 minutes.

### Example 5: Cream

**Table 5**

| Cream | | | |
|---|---|---|---|
| **Phase** | **INCI Name (Trade Name)** | % | **500 G** |
| A | Water | 27.60 | 138.00 |
| | Acrylates/C10-30 alkyl acrylate crosspolymer (Pemulen, Noveon) | 0.30 | 1.50 |
| | | | |
| B | Propylene glycol | 5.00 | 25.00 |
| | Pontylene glycol | 20.00 | 100.00 |
| | Butylene glycol | 20.00 | 100.00 |
| | Hydrocortisone | 1.00 | 5.00 |
| | | | |
| C | Dicaprytyl ether (Cetiol OE) | 3.00 | 15.00 |
| | C12-15 Alkyl benzoate | 3.00 | 15.00 |
| | Octyl palmitate | 1.00 | 5.00 |
| | Isononyl isononaoate | 3.00 | 15.00 |
| | Arachidyl alcohol and behenyl alcohol and arachidyl glucoside (Montanov 202) | 2.50 | 12.50 |
| | Behenyl alcohol | 0.50 | 2.50 |
| | | | |
| D | Triethanolamine | 0.10 | 0.50 |
| | Water | 5.00 | 25.00 |
| | | | |
| E | Glyceryl polyacrylate (Hispagel 200) | 8.00 | 40.00 |
| | | | |
| | **Total** | 100.00 | 500.00 |

The batch amount of water was weighed and added to the main beaker. Pemulen™ was added to the water and homogenized until it was dispersed. The ingredients of phase B were weighed and combined in a separate container and mixed at 50°C until clear, and then added to the main beaker while mixing. Mixing was continued until homogeneous. The ingredients of each of phases C, D and E were weighed and combined in three separate beakers. Phase C ingredients were heated to 80°C and then added to the main beaker (phases A and B) that was also heated to 80°C. The resultant mixture of phases A, B and C was homogenized for 3 minutes, followed by addition of phase D and homogenization for an additional 2 minutes. The resultant batch was cooled to 55°C, followed by addition of phase E, and homogenizing for an additional 3 minutes.

### Example 6: Gel

**Table 6**

| Hydrocortisone gel | | | |
|---|---|---|---|
| **Phase** | **INCI Name (Trade Name)** | **%** | **500 G** |
| A | Water | 40.60 | 203.00 |
| | Carbomer (carbopol 980) | 0.30 | 1.50 |
| | | | |
| B | Propylene glycol | 5.00 | 25.00 |
| | Pentylene glycol | 20.00 | 100.00 |
| | Butylene glycol | 20.00 | 100.00 |
| | Hydrocortisone | 1.00 | 5.00 |
| | | | |
| C | Triethanolamine | 0.10 | 0.50 |
| | Water | 5.00 | 25.00 |
| | | | |
| D | Glyceryl polyacrylate (Hispagel 200) | 8.00 | 40.00 |
| | | | |
| | **Total** | 100.00 | 500.00 |

The batch amount of water was weighed and added to the main beaker. The ingredients of phase B were weighed and combined in a separate container and mixed at 50°C until clear. Phase B ingredients were cooled to room temperature and added to the main beaker. Carbopol™ was sprinkled in the beaker while mixing. Mixing was continued until all dispersed. All ingredients of phases C and D were weighed and combined each in separate beakers. Phase C was added to the batch while mixing. Mixing was continued until it was clear. Phase D was added to the batch while mixing, which was continued until all clear.

### Example 7: Comparison of Release of Hydrocortisone from Embodiment of present invention and various Commercial Products

The materials used included sodium hydroxide and potassium phosphate monobasic (Malinckrodt Baker, Inc., Paris, KT) ; hydrocortisone USP (Pharmacia & Upjohn, Kalamazoo, MI); acetonitrile HPLC grade (Burdick & Jackson, Muskegon, MI); cellulose nitrate membrane filters 0.45 µm x 25mm (Whatman, Maidstone, England); PVDF syringe filters 0.45 µm (Whatman, Clifton, NJ).

The gel described in Example 6 (the gel of the present invention) was tested against competitive products. Competitor products tested included Cortaid 1% Hydrocortisone Anti-Itch Cream (Pharmacia, Peapack, NJ) ; Rite Aid Hydrocortisone Cream 1% Plus 12 Moisturizers (Rite Aid, Harrisburg, PA) ; Cortizone 10 1% Hydrocortisone Anti-Itch Ointment and Cortizone 10 1% Hydrocortisone Anti-Itch Cream (Pfizer, Morris Plains, NJ).

Dissolution testing was performed using a Van Kel VK 7000 dissolution instrument, a Van Kel VK 750D water bath/circulator and a Van Kel VK 8000 autosa pler (Varian, Cary, NC). Formulations were run in triplicates using one-liter vessels with a 7.0 phosphate buffer saline solution as the receptor phase. Paddles mixed at 100 RPM and 0.45 urn pore size cellulose nitrate membrane filters were used. The filters were soaked in phosphate buffer saline solution for 10 minutes before dissolution testing. A constant temperature of 32°C was maintained. Samples were taken at 0.5, 1, 2, 3, 4, and 6 hours for fast release products and 1, 2, 4, 6, 12, and 24 hours for slow release products. Sample volumes of 10 ml were taken directly into disposable test tubes and concentrations were calculated with a dilution factor to account for drug and volume taken from the vessels during previous sampling.

Analysis was conducted using two assay methods. The first method, UV-VIS, entailed use of an Agilent 8453 (Agilent Technologies, Wilmington, DE) UV-Vis spectrophotometer. Absorbance of hydrocortisone was read at 248 nm. HPLC analysis of samples of current marketed products was done using High Performance Liquid Chromatography (HPLC). A Perkin Elmer Series 200 HPLC apparatus (Perkin Elmer, Norwalk, CT) was used with the detector set at 248 nm. Samples were filtered prior to analysis with 0.45 µm PVDF syringe filters. The mobile phase at time of injection was 30:70 acetonitrile to water. A gradient, increasing the acetonitrile to water ratio to 70:30, was then used to cleanse the column between samples and prepare for the next injection. The injection volume was 10 µL. A Phenomenex Prodigy 0DS2 C18 4.6 mm x 25 cm particle size 5 µm column (Phenomenex, Torrance, CA) was used at 30° C. The flow rate was 1 mL/min. The retention time of Hydrocortisone was 10.7 minutes.

The results are shown in Fig. 1. The release of hydrocortisone from the gel of the present invention was about 100 times greater than the release of hydrocortisone from the Commercial products, none of which contain pentylene glycol. The results show that compositions of the present invention provide greater availability of the active agent to penetrate the affected area on the skin or scalp, and thus provide greater bioavailability of the active agent.

## Claims

1. Use of pentylene glycol as solvent for hydrocortisone or derivatives thereof chosen amongst hydrocortisone 21-acetate, hydrocortisone 21-benzadac, hydrocortisone 21-cyclopentylpropionate, hydrocortisone 21-hemisuccinate, hydrocortisone 21-acetate 17-propionate, hydrocortisone 17-butyrate, hydrocortisone 17-valerate and hydrocortisone 17-butyrate 21-propionate, in a cosmetic or dermatological composition for topical use, wherein the composition comprises at least one solvent other than pentylene glycol.

2. Use of claim 1, wherein said at least one solvent other than pentylene glycol is present at 20 to 95% by weight less than if pentylene glycol were not present.

3. The use of claim 1 or 2, wherein the hydrocortisone derivative is hydrocortisone 21-acetate 17-propionate.

4. The use of anyone of the preceding claims, wherein said at least one solvent comprises a glycol.

5. The use of anyone of the preceding claims, wherein said at least one solvent is butylene glycol.

6. The use of anyone of claims 1 to 4, wherein said at least one solvent is propylene glycol.

7. The use of anyone of claims 1 to 4, wherein said at least one solvent comprises butylene glycol and propylene glycol.

8. The use of anyone of the preceding claims, wherein amount of pentylene glycol is 5 to 70% by weight of said composition.

9. The use of anyone of the preceding claims, wherein amount of said at least one solvent other than pentylene glycol is 5% to 50% by weight of said composition.

10. The use of anyone of the preceding claims, wherein the said composition is an emulsion.

11. The use of anyone of claim 1 to 9, wherein the said composition is a gel.

12. The use of anyone of claim 1 to 9, wherein the said composition is an ointment.

13. The use of anyone of claim 1 to 9, wherein the said composition is a lotion.

14. A cosmetic or dermatological composition for topical use comprising at least hydrocortisone or derivatives thereof chosen amongst hydrocortisone 21-acetate, hydrocortisone 21-benzadac, hydrocortisone 21-cyclopentylpropionate, hydrocortisone 21-hemisuccinate, hydrocortisone 21-acetate 17-propionate, hydrocortisone 17-butyrate, hydrocortisone 17-valerate and hydrocortisone 17-butyrate 21-propionate, pentylene glycol as solvent of said corticosteroid and at least one solvent other than pentylene glycol, the amount of said hydrocortisone or derivatives thereof being of between 0.5% and 12% by weight of said composition.

15. The composition of preceding claim, wherein the at least one solvent other than pentylene glycol is chosen amongst propylene glycol and/or butylene glycol.

16. The composition of claim 14 or 15, wherein the corticosteroid is hydrocortisone 21-acetate 17-propionate.

17. The composition of anyone of claim 14 to 16, comprising butylene gycol and propylene glycol.

18. The composition of anyone of claim 14 to 17, wherein amount of pentylene glycol is 5 to 70% by weight of said composition.

19. The composition of anyone of claim 14 to 18, wherein amount of said at least one solvent other than pentylene glycol is 5 to 50% by weight of the composition.

20. The composition of anyone of claim 14 to 19, wherein amount of pentylene glycol is 5 to 25% by weight of said composition and amount of said at least one solvent other than pentylene glycol is 10% to 70% by weight of said composition.

21. The composition of anyone of claim 14 to 20, which is an emulsion.

22. The composition of anyone of claim 14 to 20, which is a gel.

23. The composition of anyone of claim 14 to 20, which is an ointment.

24. The composition of anyone of claim 14 to 20, which is a lotion.

25. The composition of anyone of claim 14 to 20, which is a shampoo.

26. A method of formulating hydrocortisone or derivatives thereof chosen amongst hydrocortisone 21-acetate, hydrocortisone 21-benzadac, hydrocortisone 21-cyclopentylpropionate, hydrocortisone 21-hemisuccinate, hydrocortisone 21-acetate 17-propionate, hydrocortisone 17-butyrate, hydrocortisone 17-valerate and hydrocortisone 17-butyrate 21-propionate for use in a cosmetic or dermatological composition, comprising mixing said hydrocortisone or derivatives thereof and a solvent for hydrocortisone or derivatives thereof comprising pentylene glycol and at least one other solvent than pentylene glycol, the amount of said hydrocortisone or derivatives thereof being of between 0.5% and 12% by weight of said composition.

## Patentansprüche

1. Verwendung von Pentylenglykol als Lösungsmittel für Hydrocortison oder dessen Derivaten, gewählt unter Hydrocortison-21-acetat, Hydrocortison-21-benzadac, Hydrocortison-21 -cyclopentylpropionat, Hydrocortison-21-hemisuccinat, Hydrocortison-21-acetat-17-propionat, Hydrocortison-17-butyrat, Hydrocortison-17-valerat und Hydrocortison-17-butyrat-21-propionat, in einer kosmetischen oder dermatologischen Zusammensetzung zur topischen Anwendung, wobei die Zusammensetzung mindestens ein anderes Lösungsmittel als Pentylenglykol umfasst.

2. Verwendung nach Anspruch 1, wobei das mindestens eine andere Lösungsmittel als Pentylenglykol zu 20 bis 95 Gew.-% weniger vorhanden ist, als wenn Pentylenglykol nicht vorhanden wäre.

3. Verwendung nach Anspruch 1 oder 2, wobei das Hydrocortison-Derivat Hydrocortison-21-acetat-17-propionat ist.

4. Verwendung nach einem der vorhergehenden Ansprüche, wobei das mindestens eine andere Lösungsmittel ein Glykol umfasst.

5. Verwendung nach einem der vorhergehenden Ansprüche, wobei das mindestens eine Lösungsmittel Butylenglykol ist.

6. Verwendung nach einem der Ansprüche 1 bis 4, wobei das mindestens eine Lösungsmittel Propylenglykol ist.

7. Verwendung nach einem der Ansprüche 1 bis 4, wobei das mindestens eine Lösungsmittel Butylenglykol und Propylenglykol umfasst.

8. Verwendung nach einem der vorhergehenden Ansprüche, wobei die Menge an Pentylenglykol 5 bis 70 Gew.-% der Zusammensetzung darstellt.

9. Verwendung nach einem der vorhergehenden Ansprüche, wobei die Menge an dem mindestens einen anderen Lösungsmittel als Pentylenglykol 5 bis 50 Gew.-% der Zusammensetzung darstellt.

10. Verwendung nach einem der vorhergehenden Ansprüche, wobei die Zusammensetzung eine Emulsion ist.

11. Verwendung nach einem der Ansprüche 1 bis 9, wobei die Zusammensetzung ein Gel ist.

12. Verwendung nach einem der Ansprüche 1 bis 9, wobei die Zusammensetzung eine Salbe ist.

13. Verwendung nach einem der Ansprüche 1 bis 9, wobei die Zusammensetzung eine Lotion ist.

14. Kosmetische oder dermatologische Zusammensetzung zur topischen Anwendung, umfassend mindestens Hydrocortison oder dessen Derivate, gewählt unter Hydrocortison-21-acetat, Hydrocortison-21-benzadac, Hydrocortison-21-cyclopentylpropionat, Hydrocortison-21-hemisuccinat, Hydrocortison-21-acetat-17-propionat, Hydrocortison-17-butyrat, Hydrocortison-17-valerat und Hydrocortison-17-butyrat-21-propionat, Pentylenglykol als Lösungsmittel dieses Kortikosteroids und mindestens ein anderes Lösungsmittel als Pentylenglykol, wobei die Menge an Hydrocortison oder dessen Derivate zwischen 0,5 und 12 Gew.-% der Zusammensetzung darstellt.

15. Zusammensetzung nach dem vorhergehenden Anspruch, wobei das mindestens eine andere Lösungsmittel als Pentylenglykol unter Propylenglykol und/oder Butylenglykol gewählt wird.

16. Zusammensetzung nach Anspruch 14 oder 15, wobei das Kortikosteroid Hydrocortison-21-acetat-17-propionat ist.

17. Zusammensetzung nach einem der Ansprüche 14 bis 16, umfassend Butylenglykol und Proylenglykol.

18. Zusammensetzung nach einem der Ansprüche 14 bis 17, wobei die Menge an Pentylenglykol 5 bis 70 Gew.-% der Zusammensetzung darstellt.

19. Zusammensetzung nach einem der Ansprüche 14 bis 18, wobei die Menge an dem mindestens einen anderen Lösungsmittel als Pentylenglykol 5 bis 50 Gew.-% der Zusammensetzung darstellt.

20. Zusammensetzung nach einem der Ansprüche 14 bis 19, wobei die Menge an Pentylenglykol 5 bis 25 Gew.-% der Zusammensetzung darstellt und die Menge an dem mindestens einen anderen Lösungsmittel als Pentylenglykol 10 bis 70 Gew.-% der Zusammensetzung darstellt.

21. Zusammensetzung nach einem der Ansprüche 14 bis 20, wobei die Zusammensetzung eine Emulsion ist.

22. Zusammensetzung nach einem der Ansprüche 14 bis 20, wobei die Zusammensetzung ein Gel ist.

23. Zusammensetzung nach einem der Ansprüche 14 bis 20, wobei die Zusammensetzung eine Salbe ist.

24. Zusammensetzung nach einem der Ansprüche 14 bis 20, wobei die Zusammensetzung eine Lotion ist.

25. Zusammensetzung nach einem der Ansprüche 14 bis 20, wobei die Zusammensetzung ein Shampoo ist.

26. Verfahren zur Formulierung von Hydrocortison oder dessen Derivaten, gewählt unter Hydrocortison-21-acetat, Hydrocortison-21-benzadac, Hydrocortison-21-cyclopentylpropionat, Hydrocortison-21-hemisuccinat, Hydrocortison-21-acetat-17-propionat, Hydrocortison-17-butyrat, Hydrocortison-17-valerat und Hydrocortison-17-butyrat-21-propionat zur Verwendung in einer kosmetischen oder dermatologischen Zusammensetzung, umfassend das Mischen des Hydrocortisons oder dessen Derivate mit einem Lösungsmittel für Hydrocortison oder dessen Derivate, umfassend Pentylenglykol und mindestens ein anderes Lösungsmittel als Pentylenglykol, wobei die Menge des Hydrocortisons oder dessen Derivate 0,5 bis 12 Gew.-% der Zusammensetzung darstellt.

## Revendications

1. Utilisation de pentylène glycol en tant que solvant pour l'hydrocortisone ou ses dérivés choisis parmi le 21-acétate d'hydrocortisone, le 21-benzadac d'hydrocortisone, le 21-cyclopentylpropionate d'hydrocortisone, le 21-hémisuccinate d'hydrocortisone, le 21-acétate-17-propionate d'hydrocortisone, le 17-butyrate d'hydrocortisone, le 17-valérate d'hydrocortisone, et le 17-butyrate-21-propionate d'hydrocortisone, dans une composition cosmétique ou dermatologique à usage topique, dans laquelle la composition comprend au moins un solvant autre que le pentylène glycol.

2. Utilisation selon la revendication 1, dans laquelle ledit au moins un solvant autre que le pentylène glycol est présent de 20 à 95 % en poids de moins que si le pentylène glycol n'était pas présent.

3. Utilisation selon la revendication 1 ou 2, dans laquelle le dérivé d'hydrocortisone est le 21-acétate-17-propionate d'hydrocortisone.

4. Utilisation selon l'une quelconque des revendications précédentes, dans laquelle ledit au moins un solvant comprend un glycol.

5. Utilisation selon l'une quelconque des revendications précédentes, dans laquelle ledit au moins un solvant est le butylène glycol.

6. Utilisation selon l'une quelconque des revendications 1 à 4, dans laquelle ledit au moins un solvant est le propylène glycol.

7. Utilisation selon l'une quelconque des revendications 1 à 4, dans laquelle ledit au moins un solvant comprend du butylène glycol et du propylène glycol.

8. Utilisation selon l'une quelconque des revendications précédentes, dans laquelle la quantité de pentylène glycol est de 5 à 70 % en poids de ladite composition.

9. Utilisation selon l'une quelconque des revendications précédentes, dans laquelle la quantité dudit au moins un solvant autre que le pentylène glycol est de 5 % à 50 % en poids de ladite composition.

10. Utilisation selon l'une quelconque des revendications précédentes, dans laquelle ladite composition est une émulsion.

11. Utilisation selon l'une quelconque des revendications 1 à 9, dans laquelle ladite composition est un gel.

12. Utilisation selon l'une quelconque des revendications 1 à 9, dans laquelle ladite composition est une pommade.

13. Utilisation selon l'une quelconque des revendications 1 à 9, dans laquelle ladite composition est une lotion.

14. Composition cosmétique ou dermatologique à usage topique comprenant au moins de l'hydrocortisone ou ses dérivés choisis parmi le 21-acétate d'hydrocortisone, le 21-benzadac d'hydrocortisone, le 21-cyclopentylpropionate d'hydrocortisone, le 21-hémisuccinate d'hydrocortisone, le 21-acétate-17-propionate d'hydrocortisone, le 17-butyrate d'hydrocortisone, le 17-valérate d'hydrocortisone, et le 17-butyrate-21-propionate d'hydrocortisone, du pentylène glycol en tant que solvant dudit corticostéroïde, et au moins un solvant autre que le pentylène glycol, la quantité de ladite hydrocortisone ou de ses dérivés étant comprise entre 0,5 % et 12 % en poids de ladite composition.

15. Composition selon la revendication précédente, dans laquelle ledit au moins un solvant autre que le pentylène glycol est choisi parmi le propylène glycol et/ou le butylène glycol.

16. Composition selon la revendication 14 ou 15, dans laquelle le corticostéroïde est le 21-acétate-17-propionate d'hydrocortisone.

17. Composition selon l'une quelconque des revendications 14 à 16, comprenant du butylène glycol et du propylène glycol.

18. Composition selon l'une quelconque des revendications 14 à 17, dans laquelle la quantité de pentylène glycol est de 5 à 70 % en poids de ladite composition.

19. Composition selon l'une quelconque des revendications 14 à 18, dans laquelle la quantité dudit au moins un solvant autre que le pentylène glycol est de 5 à 50 % en poids de la composition.

20. Composition selon l'une quelconque des revendications 14 à 19, dans laquelle la quantité de pentylène glycol est de 5 à 25 % en poids de ladite composition et la quantité dudit au moins un solvant autre que le pentylène glycol est de 10 % à 70 % en poids de ladite composition.

21. Composition selon l'une quelconque des revendications 14 à 20, qui est une émulsion.

22. Composition selon l'une quelconque des revendications 14 à 20, qui est un gel.

23. Composition selon l'une quelconque des revendications 14 à 20, qui est une pommade.

24. Composition selon l'une quelconque des revendications 14 à 20, qui est une lotion.

25. Composition selon l'une quelconque des revendications 14 à 20, qui est un shampooing.

26. Procédé pour formuler de l'hydrocortisone ou ses dérivés choisis parmi le 21-acétate d'hydrocortisone, le 21-benzadac d'hydrocortisone, le 21-cyclopentylpropionate d'hydrocortisone, le 21-hémisuccinate d'hydrocortisone, le 21-acétate-17-propionate d'hydrocortisone, le 17-butyrate d'hydrocortisone, le 17-valérate d'hydrocortisone, et le 17-butyrate-21-propionate d'hydrocortisone, pour son utilisation dans une composition cosmétique ou dermatologique, comprenant le mélange de ladite hydrocortisone ou de ses dérivés et d'un solvant pour l'hydrocortisone ou ses dérivés comprenant du pentylène glycol et au moins un solvant autre que le pentylène glycol, la quantité de ladite hydrocortisone ou de ses dérivés étant comprise entre 0,5 % et 12 % en poids de ladite composition.
